# EUROPEAN PATENT SPECIFICATION

(11) **EP 1 507 510 B1**
(45) Date of publication and mention of the grant of the patent: **31.01.2007**
(21) Application number: 03732458.9
(22) Date of filing: 22.05.2003
(51) Int. Cl.: A61Q 19/08, A61K 8/362

(54) **COSMETIC COMPOSITIONS WITH AMMONIUM MALONATES**
KOSMETISCHE ZUSAMMENSETZUNGEN MIT AMMONIUMMALONATEN
COMPOSITIONS COSMETIQUES CONTENANT DES MALONATES D'AMMONIUM

(30) Priority: 29.05.2002 US 383837 P; 06.09.2002 US 408580 P
(43) Date of publication of application: 23.02.2005
(73) Proprietor: UNILEVER PLC, London EC4P 4BQ (GB); UNILEVER N.V., 3013 AL Rotterdam (NL)
(72) Inventor: FARYNIARZ, Joseph Raymond, Trumbull, CT 06611 (US); JOHNSON, Anthony William, Trumbull, CT 06611 (US); SUARES, Alan Joseph, Trumbull, CT 06611 (US); CHENEY, Michael Charles, Trumbull, CT 06611 (US)
(74) Representative: Acham, Nicholas Clive
(86) International application number: PCT/EP2003/005462
(87) International publication number: WO 2003/099251

(56) References cited:
- EP-A- 0 396 857
- EP-A- 0 969 089
- EP-A- 1 090 630
- EP-A- 1 192 940
- WO-A-00/61107
- WO-A-97/33560
- US-A- 5 578 641
- US-A- 5 643 586
- DATABASE EMBASE [Online] ELSEVIER SCIENCE PUBLISHERS, AMSTERDAM, NL; 1979 ISHIHARA N; IKEDA M: "Effects of solvents and solutes on the percutaneous absorption of m-dinitrobenzene" Database accession no. EMB-1979259894 XP002249478
- PATENT ABSTRACTS OF JAPAN vol. 1998, no. 11, 30 September 1998 (1998-09-30) & JP 10 175844 A (SHISEIDO CO LTD), 30 June 1998 (1998-06-30)

## Description

The invention concerns cosmetic compositions containing ammonium malonates which combat the signs of skin aging.

A soft, supple and flexible skin has a marked cosmetic appeal, and is an attribute of normal functioning epidermis. As human skin ages with advancing years, the epidermis can become folded, ridged or furrowed to form wrinkles. These signal loss of youthful appearance and herald the transition to old age. Exposure to excessive doses of sunlight accelerates the transition process. Also, the outer layer of the epidermis known as the stratum corneum can become dry and flaky following exposure to cold weather or excessive contact with detergents or solvents. Loss of skin moisture thereby results, and the skin begins to lose the soft, supple and flexible characteristics.

Emollients such as fats, phospholipids and sterols have in the past been used to soften wrinkled or dry skin. These emollients are only partially effective as a remedy for skin in poor condition.

The use of hydroxy carboxylic acids for enhancing the quality of human skin has been known for some time. There is no doubt that alpha-hydroxy carboxylic acids are effective much beyond the common emollients.

U.S. Patent 4,424,234 (Alderson et al.) discloses skin treatment compositions incorporating alpha-hydroxycaproic acid and alpha-hydroxycaprylic acid or mixtures thereof in compositions that have a pH value of less than 7, usually from 2 to 4. Yu and Van Scott have patented widely in this area. For instance, U.S. Patent 4,105,782 reports amines or ammonium salts of alpha-hydroxy carboxylic acids in the treatment of acne or dandruff. In U.S. Patent 4,105,783 and U.S. Patent 4,197,316, these compounds are suggested for the treatment of dry skin. U.S. Patent 4,234,599 discloses the use of alpha-hydroxy carboxylic acids, their esters or amine salts in the treatment of keratoses. More recently, U.S. Patent 5,091,171 focused attention on these compounds as being effective against age spots, wrinkles and aging related skin changes.

While hydroxy carboxylic acids hold much therapeutic promise, the materials have been found to irritate human skin on repeated topical applications. The irritation may range from a sensation of tingling, itching and burning to clinical signs of redness and peeling. Causes for such irritation have been linked to the lowering of pH in the stratum corneum of human skin. Low pH has been suggested as provoking disturbances in intercorneocyte bondings resulting in adverse skin reactions, specially in some individuals with sensitive skin.

Organic acids other than alpha-hydroxy functionalized have been disclosed in the cosmetic literature. For instance, U.S. Patent 5,641,495 (Jokura et al.) discloses in combination a ceramide or pseudoceramide, a dicarboxylic acid and a salt of a dicarboxylic acid. The examples illustrate sodium and potassium salts of succinic acid.

Lower molecular weight dicarboxylic acids such as malonic may also be utilized.

Although excellent moisturization and little accompanying irritation occurs, there is no suggestion that this system combats signs of aging such as advent of fine lines and wrinkles. Improvements in the general anti-ageing technology of skin remains as an unfulfilled need of the consumer.

Accordingly, it is an advantage of the present invention to be able to provide new cosmetic ingredients in compositions which are effective at controlling and even eliminating the signs of aging, particularly fine lines, wrinkles, sagging skin, poor tone and age spots.

In a first aspect of the invention, there is provided use of a cosmetic composition for controlling signs of ageing selected from the group consisting of fine lines, wrinkles, sagging skin, poor tone and age spots. The composition includes:
(i) from 0.1% to 30% by weight of a salt which is an ammonia neutralized malonic acid;
(ii) from 1% to 99.9% by weight of a cosmetically acceptable carrier;
wherein the composition has a pH ranging from 3 to 6.5.

We have now found a class of salts which are at least as effective as alpha-hydroxy carboxylic acids. These salts are based on malonic acid neutralized with ammonia. These salts may either be the half or fully neutralized malonate salts or combinations thereof as represented by general formulas (I) and (II): 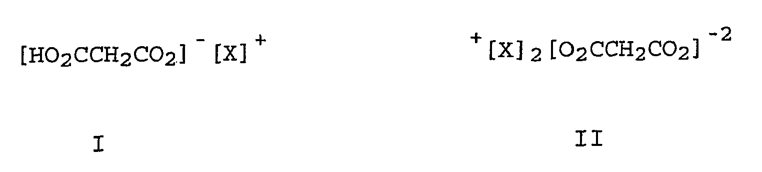 wherein X is a protonated ammonia.

Amounts of the ammonia neutralized malonic acid salt may range from 0.1% to 30% by weight of the cosmetic composition.

The present invention can utilize as the active ingredient salt I, salt II or mixtures of these salts. When mixtures are present the molar ratio of mono-salt I to di-salt II may range from about 1000:1 to about 1:1000, preferably from about 10:1 to about 1:500, more preferably from about 2:1 to about 1:200, optimally from about 1:1 to about 1:20.

Compositions of this invention may have a pH ranging from 3 to 6.5.

Compositions of this invention will also include a cosmetically acceptable carrier. Amounts of the carrier may range from 1% to 99.9%, preferably from about 70% to about 95%, optimally from about 80% to about 90%. Among the useful carriers are water, emollients, fatty acids, fatty alcohols, humectants, thickeners and combinations thereof. The carrier may be aqueous, anhydrous or an emulsion. Preferably the compositions are aqueous, especially water and oil emulsions of the W/O or O/W variety. Water when present may be in amounts ranging from about 5% to about 95%, preferably from about 20% to about 70%, optimally from about 35% to about 60% by weight.

Emollient materials may serve as cosmetically acceptable carriers. These may be in the form of silicone oils, synthetic esters and hydrocarbons. Amounts of the emollients may range anywhere from about 0.1% to about 95%, preferably between about 1% and about 50% by weight.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non-volatile silicone oils useful as an emollient material include polyalkyl siloxanes, polyalkylaryl siloxanes and polyether siloxane copolymers. The essentially nonvolatile polyalkyl siloxanes useful herein include, for example, polydimethyl siloxanes with viscosities of from about 5 x 10⁻⁶ to 0.1 m²/s at 25°C. Among the preferred non-volatile emollients useful in the present compositions are the polydimethyl siloxanes having viscosities from about 1 x 10⁻⁵ to about 4 x 10⁻⁴ m² /s at 25°C.

Another class of non-volatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Among the suitable ester emollients are:
(1) Alkenyl or alkyl esters of fatty acids having 10 to 20 carbon atoms. Examples thereof include isoarachidyl neopentanoate, isononyl isonanonoate, oleyl myristate, oleyl stearate, and oleyl oleate.
(2) Ether-esters such as fatty acid esters of ethoxylated fatty alcohols.
(3) Polyhydric alcohol esters. Ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of C₁-C₃₀ alcohols.
(4) Wax esters such as beeswax, spermaceti wax and tribehenin wax.
(5) Sterols esters, of which cholesterol fatty acid esters are examples thereof.
(6) Sugar ester of fatty acids such as sucrose polybehenate and sucrose polycottonseedate.

Hydrocarbons which are suitable cosmetically acceptable carriers include petrolatum, mineral oil, C₁₁-C₁₃ isoparaffins, polyalphaolefins, and especially isohexadecane, available commercially as Permethyl 101A from Presperse Inc.

Fatty acids having from 10 to 30 carbon atoms may also be suitable as cosmetically acceptable carriers. Illustrative of this category are pelargonic, lauric, myristic, palmitic, stearic, isostearic, hydroxystearic, oleic, linoleic, ricinoleic, arachidic, behenic and erucic acids.

Fatty alcohols having from 10 to 30 carbon atoms are another useful category of cosmetically acceptable carrier. Illustrative of this category are stearyl alcohol, lauryl alcohol, myristyl alcohol and cetyl alcohol.

Humectants of the polyhydric alcohol-type can be employed as cosmetically acceptable carriers. Typical polyhydric alcohols include glycerol, polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5% to 50%, preferably between 1% and 15% by weight of the composition.

Thickeners can be utilized as part of the cosmetically acceptable carrier of compositions according to the present invention. Typical thickeners include crosslinked acrylates (e.g. Carbopol 982®), hydrophobically-modified acrylates (e.g. Carbopol 1382®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, and silicates such as magnesium aluminum silicate (Veegum®). Amounts of the thickener may range from 0.0001% to 10%, usually from 0.001% to 1%, optimally from 0.01% to 0.5% by weight.

Cosmetic compositions of the present invention may be in any form. These forms may include lotions, creams, roll-on formulations, sticks, mousses, aerosol and non-aerosol sprays and pad-applied formulations.

Surfactants may also be present in cosmetic compositions of the present invention. Total concentration of the surfactant when present may range from about 0.1% to about 40%, preferably from about 1% to about 20%, optimally from about 1% to about 5% by weight of the composition. The surfactant may be selected from the group consisting of anionic, nonionic, cationic and amphoteric actives.

Particularly preferred nonionic surfactants are those with a C₁₀-C₂₀ fatty alcohol or acid hydrophobe condensed with from 2 to 100 moles of ethylene oxide or propylene oxide per mole of hydrophobe; C₂-C₁₀ alkyl phenols condensed with from 2 to 20 moles of alkylene oxide; mono- and di-fatty acid esters of ethylene glycol; fatty acid monoglyceride; sorbitan, mono- and di- C₈-C₂₀ fatty acids; and polyoxyethylene sorbitan as well as combinations thereof. Alkyl polyglycosides and saccharide fatty amides (e.g. methyl gluconamides) are also suitable nonionic surfactants.

Preferred anionic surfactants include soap, alkyl ether sulfates and sulfonates, alkyl sulfates and sulfonates, alkylbenzene sulfonates, alkyl and dialkyl sulfosuccinates, C₈-C₂₀ acyl isethionate, C₈-C₂₀ alkyl ether phosphates, C₈-C₂₀ sarcosinates and combinations thereof.

Sunscreen actives may also be included in compositions of the present invention. Particularly preferred are such materials as ethylhexyl p-methoxycinnamate, available as Parsol MCX®, Avobenzene, available as Parsol 1789® and benzophenone-3, also known as Oxybenzone. Inorganic sunscreen actives may be employed such as microfine titanium dioxide, zinc oxide, polyethylene and various other polymers. Amounts of the sunscreen agents when present may generally range from 0.1% to 30%, preferably from 2% to 20%, optimally from 4% to 10% by weight.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

Compositions of the present invention may include vitamins. Illustrative vitamins are Vitamin A (retinol), Vitamin B₂, Vitamin B₆, Vitamin C, Vitamin E and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001% to 10%, preferably from 0.01% to 1%, optimally from 0.1% to 0.5% by weight.

Another type of useful substance can be that of an enzyme such as oxidases, proteases, lipases and combinations.

Particularly preferred is superoxide dismutase, commercially available as Biocell SOD from the Brooks Company, USA.

Skin lightening compounds may be included in the compositions of the invention. Illustrative substances are placental extract, lactic acid, niacinamide, arbutin, kojic acid, ferulic acid, resorcinol and derivatives including 4-substituted resorcinols and combinations thereof. Amounts of these agents may range from about 0.1% to about 10%, preferably from about 0.5% to about 2% by weight of the compositions.

Desquamation promoters may be present. Illustrative are the alpha-hydroxycarboxylic acids and beta-hydroxycarboxylic acids. The term "acid" is meant to include not only the free acid but also salts and C₁-C₃₀ alkyl or aryl esters thereof and lactones generated from removal of water to form cyclic or linear lactone structures. Representative acids are glycolic, lactic and malic acids. Salicylic acid is representative of the beta-hydroxycarboxylic acids. Amounts of these materials when present may range from about 0.1% to about 15% by weight of the composition.

A variety of herbal extracts may optionally be included in compositions of this invention. Illustrative are green tea, chamomile, licorice and extract combinations thereof. The extracts may either be water soluble or water-insoluble carried in a solvent which respectively is hydrophilic or hydrophobic. Water and ethanol are the preferred extract solvents.

Also included may be such materials as lipoic acid, retinoxytrimethylsilane (available from Clariant Corp. under the Silcare 1M-75 trademark), ceramides (including Ceramide 1, Ceramide 3, Ceramide 3B and Ceramide 6), dehydroepiandrosterone (DHEA) and combinations thereof. Amounts of these materials may range from about 0.000001% to about 10%, preferably from about 0.0001% to about 1% by weight.

Colorants, fragrances, opacifiers and abrasives may also be included in compositions of the present invention. Each of these substances may range from about 0.05% to about 5%, preferably between 0.1% and 3% by weight.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

Except in the operating and comparative examples, or where otherwise explicitly indicated, all numbers in this description indicating amounts of material ought to be understood as modified by the word "about".

### Examples

The following examples will more fully illustrate the embodiments of this invention. All parts, percentages and proportions referred to herein and in the appended claims are by weight unless otherwise illustrated.

### Example 1

A clinical study was conducted to compare ammonium malonate to ammonium glycolate as active cosmetic ingredients. The base formula for the comparative experiments is outlined under Table I.

**TABLE I**

| INGREDIENT | WEIGHT % |
|---|---|
| PHASE A | |
| Water | Balance |
| Disodium EDTA | 0.05 |
| Methyl Paraben | 0.15 |
| Magnesium Aluminum Silicate | 0.60 |
| Triethanolamine | 1.20 |

| PHASE B | |
|---|---|
| Xanthan Gum | 0.20 |
| Natrosol® 250HHR (ethyl cellulose) | 0.50 |
| Butylene Glycol | 3.00 |
| Glycerin | 2.00 |

| PHASE C | |
|---|---|
| Sodium Stearoyl Lactylate | 0.10 |
| Glycerol Monostearate | 1.50 |
| Stearyl Alcohol | 1.50 |
| Isostearyl Palmitate | 3.00 |
| Silicone Fluid | 1.00 |
| Cholesterol | 0.25 |
| Sorbitan Stearate | 1.00 |
| Butylated Hydroxy Toluene | 0.05 |
| Vitamin E Acetate | 0.01 |
| PEG-100 Stearate | 2.00 |
| Stearic Acid | 3.00 |
| Propyl Paraben | 0.10 |
| Parsol MCX® | 2.00 |
| Caprylic/Capric Triglyceride | 0.50 |
| Hydroxycaprylic Acid | 0.01 |
| C12-15 Alkyl Octanoate | 3.00 |

| PHASE D | |
|---|---|
| Active | |

| PHASE E | |
|---|---|
| Vitamin A Palmitate | 0.10 |
| Bisabolol | 0.01 |
| Vitamin A Acetate | 0.01 |
| Fragrance | 0.03 |
| Retinol 50C | 0.02 |

The total formulations with ammonium glycolate and ammonium malonate active are identified in the Tables below as "PADC" and "Ammonium". The PADC product is a state of the art alpha hydroxy acid formula which is currently in the market. This formula contains 8% Glycolic acid or 0.1053 equivalents, neutralized with 2.4% ammonia hydroxide which is 0.0395 equivalents, resulting in a final formula pH of 3.8. The ammonium formula contains 5.04% malonic acid or 0.0969 equivalents, neutralized with 1.87% ammonia which is 0.0311 equivalents, resulting in a final formula pH of 3.6-4.0.
This resulted in Malonic acid being 31.87% neutralized by the amine.

The clinical involved 49 panellists over asix week period. Panellists were required to apply each product to one half of their face. After application, the panellists were required to answer a series of questions regarding relative effectiveness of the products.

**TABLE II**

| Products: | Week 1 (n=49) | | | Week 3 (n=49) | | | Week 6 (n=47) | | |
|---|---|---|---|---|---|---|---|---|---|
| PADC & Ammonium Malonate pH 3.6-4.0 | PADC | Ammonium | no pref | PADC | Ammonium | no pref | PADC | Ammonium | No pref |
| Absorbed more easily | 31 | 39 | 30 | 35 | 29 | 37 | 30 | 23 | 47 |
| Felt less greasy | 26 | 43 | 31 | 31 | 33 | 37 | 30 | 30 | 40 |
| Felt lighter | 33 | 39 | 28 | 35 | 35 | 31 | 32 | 34 | 34 |
| Left skin feeling softer | 24 | 22 | 54 | 29 | 18 | 53 | 30 | 15 | 55 |
| Was milder | 20 | 37 | 43 | 29 | 20 | 51 | 28 | 26 | 45 |
| Left skin feeling smoother | 24 | 22 | 54 | 29 | 12 | 59 | 32 | 13 | 55 |
| Left skin looking smoother | 22 | 15 | 63 | 27 | 14 | 59 | 28 | 19 | 53 |
| Moisturized better | 28 | 26 | 46 | 33 | 14 | 53 | 21 | 13 | 66 |
| Helped to look firmer/tighter | 26 | 22 | 52 | 22 | 16 | 61 | 26 | 15 | 59 |
| Helped to feel healthier | 19 | 13 | 69 | 22 | 10 | 67 | 23 | 13 | 64 |
| Helped to feel firmer/tighter | 24 | 24 | 52 | 22 | 25 | 53 | 25 | 19 | 57 |
| Improved skin tone better | 20 | 11 | 69 | 22 | 12 | 65 | 24 | 17 | 59 |
| Made skin look better | 20 | 17 | 63 | 25 | 10 | 65 | 25 | 13 | 62 |
| Improved condition better | 20 | 19 | 61 | 20 | 16 | 63 | 28 | 15 | 57 |
| Helped to look younger | 20 | 13 | 67 | 20 | 14 | 65 | 23 | 11 | 66 |
| Left skin more radiant | 17 | 17 | 67 | 14 | 12 | 74 | 13 | 15 | 72 |
| Was less irritating | 23 | 36 | 41 | 26 | 25 | 49 | 28 | 30 | 42 |
| Was evening out tone/texture | 15 | 9 | 76 | 18 | 10 | 71 | 21 | 9 | 70 |
| Firmed skin better | 24 | 19 | 57 | 20 | 20 | 59 | 28 | 19 | 53 |

### Overall preference

| Products: | Week 1 | | | Week 3 | | | Week 6 | | |
|---|---|---|---|---|---|---|---|---|---|
| PADC & Ammonium Malonate pH 3.6-4.0 | PADC | Ammonium | no pref | PADC | Ammonium | no pref | PADC | Ammonium | No pref |
| Overall Preference | 41 | 33 | 26 | 35 | 37 | 29 | 36 | 34 | 30 |

Based on the results of the clinical evaluations, it is evident that ammonium malonate is nearly as effective as ammonium glycolate, the well-known, but irritation inducing active, in respect of improving the general condition of skin.

Additionally, a further more extensive clinical found that ammonium malonate was considerably less irritating than the ammonium glycolate composition. Results of that study are summarized in the Table below. The products evaluated herein were identical to those fielded in the first clinical study.

### Overall Skin Problems Experienced

| Products | Week 1 | | Week 4 | | Week 8 | |
|---|---|---|---|---|---|---|
| | PADC | Ammon. Malonate | PADC | Ammon. Malonate | PADC | Ammon. Malonate |
| Yes | 24% | 11% | 10% | 5% | 9% | 5% |
| No | 76% | 89% | 90% | 95% | 91% | 95% |

The types of skin problems experienced that were recorded included any redness/splotches, pimples/breakouts/acne, tingling, burning, dryness, stinging, itching, irritation/discomfort, bumps, rash, peeling, puffiness, flaking, tightness, blotchiness, blisters/blistering and any other similar manifestation. Approximately 100 panellists were used in this clinical. It is evident that in the first week of use, the ammonium malonate is much less discomforting to the face than the ammonium glycolate.
After several weeks of use, the panellists became acclimated and the difference between the materials became less although still discernible.

### Example 2

A water-in-oil topical liquid make-up foundation utilizing the malonate salts of the present invention is described in Table IV below.

**TABLE IV**

| INGREDIENT | WEIGHT % |
|---|---|
| PHASE A | |
| Cyclomethicone | 9.25 |
| Cetyl Octanoate | 2.00 |
| Dimethicone Copolyol | 20.00 |

| PHASE B | |
|---|---|
| Talc | 3.38 |
| Pigment (iron Oxides) | 10.51 |
| Spheron L-1500 (Silica) | 0.50 |

| PHASE C | |
|---|---|
| Synthetic Wax Durachem 0602 | 0.10 |
| Arachidyl Behenate | 0.30 |

| PHASE D | |
|---|---|
| Cyclomethicone | 1.00 |
| Trihydroxystearin | 0.30 |

| PHASE E | |
|---|---|
| Laureth-7 | 0.50 |
| Propyl Paraben | 0.25 |

| PHASE F | |
|---|---|
| Fragrance | 0.05 |

| PHASE G | |
|---|---|
| Water | balance |
| Ammonium Malonate | 3.00 |
| Methyl Paraben | 0.12 |
| Propylene Glycol | 8.00 |
| Niacinamide | 4.00 |
| Glycerin | 3.00 |
| Sodium Chloride | 2.00 |
| Sodium Dehydroacetate | 0.30 |

### Example 3

Illustrated herein is a skin cream incorporating the malonate salts of the present invention.

**TABLE V**

| INGREDIENT | WEIGHT % |
|---|---|
| Glycerin | 6.93 |
| Niacinamide | 5.00 |
| Ammonium Malonate | 5.00 |
| Permethyl 101A¹ | 3.00 |
| Sepigel 305² | 2.50 |
| Q2-1403³ | 2.00 |
| Isopropyl Isostearate | 1.33 |
| Arlatone 2121⁴ | 1.00 |
| Cetyl Alcohol CO-1695 | 0.72 |
| SEFA Cottonate⁵ | 0.67 |
| Tocopherol Acetate | 0.50 |
| Panthenol | 0.50 |
| Stearyl Alcohol | 0.48 |
| Titanium Dioxide | 0.40 |
| Disodium EDTA | 0.10 |
| Glydant Plus⁶ | 0.10 |
| PEG-100 Stearate | 0.10 |
| Stearic Acid | 0.10 |
| Purified Water | Balance |

| | |
|---|---|
| ¹ Isohexadecane, Presperse Inc., South Plainfield, NJ ² Polyacrylamide(and)C13-14 Isoparaffin(and) Laureth-7, Seppic Corporation, Fairfield, NJ ³ dimethicone (and) dimethiconol, Dow Corning Corp. Midland, MI ⁴ Sorbitan Monostearate and Sucrococoate, ICI Americas Inc., Wilmington, DE ⁵ Sucrose ester of fatty acid ⁶ DMDM Hydantoin (and) Iodopropynyl Butylcarbamate, Lonza Inc., Fairlawn, NJ | |

### Example 4

Illustrative of a powdered cosmetic composition according to the present invention is the formula of Table VI.

**TABLE VI**

| INGREDIENT | WEIGHT % |
|---|---|
| Polysilicone-11 | 22.5 |
| Cyclomethicone | 59 |
| Petrolatum | 11 |
| Ammonium Malonate (50% in water) | 7 |
| Dimethicone Copolyol | 0.5 |

### Example 5

A relatively anhydrous composition according to the present invention is reported in Table VII.

**TABLE VII**

| INGREDIENT | WEIGHT % |
|---|---|
| Cyclomethicone | 80.65 |
| Dimethicone | 9.60 |
| Squalane | 6.00 |
| Isostearic Acid | 1.90 |
| Borage Seed Oil | 0.90 |
| Ammonium Malonate (50% in water) | 0.50 |
| Retinyl Palmitate | 0.25 |
| Ceramide 6 | 0.10 |
| Tocopherol | 0.10 |

### Example 6

An aerosol packaged foaming cleanser suitable for the present invention is outlined in Table VIII.

**TABLE VIII**

| INGREDIENT | WEIGHT % |
|---|---|
| Sunflower Seed Oil | 20.00 |
| Maleated Soybean Oil | 5.00 |
| Silicone Urethane | 1.00 |
| Polyglycero-4 Oleate | 1.00 |
| Sodium C14-16 Olefin Sulfonate | 15.00 |
| Sodium Lauryl Ether Sulphate (25% active) | 15.00 |
| Cocoamidopropylbetaine | 15.00 |
| DC 1784® (Silicone Emulsion 50%) | 5.00 |
| Polyquaternium-11 | 1.00 |
| Ammonium Malonate | 1.00 |
| Water | Balance |

An aerosol is prepared using 92% by weight of the concentrate in Table VIII and 8% propellant, the latter being a combination of dimethylether, isobutane and propane.

### Example 7

An adhesive cosmetic patch may also be formulated according to the present invention. An adhesive hydrogel is prepared by mixing 30 grams of 2-acrylamido-2-methylpropane sulphonic acid monomer in 20 grams distilled water and 5 grams of a 1% aqueous solution of methylene-bis-acrylamide. The solution is then activated with 0.4% magnesium persulphate catalyst. Shortly after mixing the catalyst with the hydrogel solution, 0.1 grams ammonium malonate in 5ml water is added. The resultant solution is coated onto a 50/50 blend of polypropylene and hydrophilic polyester and allowed to solidify. The resulting deposited hydrogel is warmed for 24 hours at 40°C in a hot air oven. Final water content of the hydrogel is 50%. A polystyrene backing layer is laid over the adhesive hydrogel.

### Example 8

A disposable, single use personal towelette product is described according to the present invention. A 70/30 polyester/rayon non-woven towelette is prepared with a weight of 1.8 grams and dimensions of 15 cm by 20 cm. Onto this towelette is impregnated a composition as outlined in Table IX below.

**TABLE IX**

| INGREDIENT | WEIGHT % |
|---|---|
| Ammonium Malonate | 7.50 |
| Glycerin | 2.00 |
| Hexylene Glycol | 2.00 |
| Disodium Capryl Amphodiacetate | 1.00 |
| Gluconolactone | 0.90 |
| Silicone Microemulsion | 0.85 |
| Witch Hazel | 0.50 |
| PEG-40 Hydrogenated Castor Oil | 0.50 |
| Fragrance | 0.20 |
| Vitamin E Acetate | 0.001 |
| Water | Balance |

The foregoing description and examples illustrate selected embodiments of the present invention.

## Claims

1. Use of a cosmetic composition for controlling signs of ageing selected from the group consisting of fine lines, wrinkles, sagging skin, poor tone and age spots, the composition comprising:
(i) from about 0.1% to about 30% by weight of a salt which is an ammonia neutralized malonic acid;
(ii) from about 1% to about 99.9% by weight of a cosmetically acceptable carrier;
wherein the composition has a pH ranging from 3 to 6.5.

2. Use according to claim 1 wherein the malonic acid is present as a half neutralized and a fully neutralized acid in a molar ratio ranging from about 1000:1 to about 1:1000, respectively.

3. Use according to claim 2 wherein the molar ratio is about 2:1 to about 1:200.

4. Use according to any of the preceding claims wherein the pH ranges from 3.5 to 5.5.

5. A method for controlling signs of ageing selected from the group consisting of fine lines, wrinkles, sagging skin, poor tone and age spots, comprising:
providing a cosmetic composition comprising:
(i) from about 0.1% to about 30% by weight of a salt which is an ammonia neutralized malonic acid;
(ii)from about 1% to about 99.9% by weight of a cosmetically acceptable carrier;
wherein the composition has a pH ranging from 3 to 6.5.

## Patentansprüche

1. Verwendung einer kosmetischen Zusammensetzung zum Bekämpfen der Anzeichen von Alterung, ausgewählt aus der Gruppe, bestehend aus feinen Linien, Falten, herabhängender Haut, schlechter Tönung und Altersflecken, wobei die Zusammensetzung umfasst:
(i) etwa 0,1 % bis etwa 30 Gew.-% eines Salzes, das eine mit Ammoniak neutralisierte Malonsäure darstellt;
(ii) etwa 1 % bis etwa 99,9 Gew.-% eines kosmetisch verträglichen Trägers,
wobei die Zusammensetzung einen pH-Wert im Bereich von 3 bis 6,5 aufweist.

2. Verwendung nach Anspruch 1, wobei die Malonsäure als eine halb neutralisierte und eine vollständig neutralisierte Säure in einem Molverhältnis im Bereich von etwa 1000 : 1 bzw. etwa 1 : 1000 vorliegt.

3. Verwendung nach Anspruch 2, wobei das Molverhältnis etwa 2 : 1 bis etwa 1 : 200 ist.

4. Verwendung nach einem der vorangehenden Ansprüche, wobei der pH-Wert im Bereich von 3,5 bis 5,5 liegt.

5. Verfahren zum Bekämpfen der Anzeichen von Alterung, ausgewählt aus der Gruppe, bestehend aus feinen Linien, Falten, herabhängender Haut, schlechter Tönung und Altersflecken, umfassend: Bereitstellen einer kosmetischen Zusammensetzung, umfassend:
(i) etwa 0,1 % bis etwa 30 Gew.-% eines Salzes, das eine mit Ammoniak neutralisierte Malonsäure darstellt;
(ii) etwa 1 % bis etwa 99,9 Gew.-% eines kosmetisch verträglichen Trägers,
wobei die Zusammensetzung einen pH-Wert im Bereich von 3 bis 6,5 aufweist.

## Revendications

1. Utilisation d'une composition cosmétique pour contrôler les signes du vieillissement choisis dans le groupe constitué des ridules, des rides, de l'affaissement, du manque de tonicité et des taches de vieillesse, la composition comprenant :
(i) d'environ 0,1 % à environ 30 % en poids d'un sel qui est un acide malonique neutralisé à l'ammoniac ;
(ii) d'environ 1 % à environ 99,9 % en poids d'un support cosmétiquement acceptable ;
dans laquelle la composition à un pH dans la plage de 3 à 6,5.

2. Utilisation selon la revendication 1, dans laquelle l'acide malonique est présent sous la forme d'un acide à demi neutralisé et d'un acide entièrement neutralisé dans un rapport molaire se situant dans la plage d'environ 1000 :1 à environ 1 :1000 respectivement.

3. Utilisation selon la revendication 2, dans laquelle le rapport molaire est d'environ 2 :1 à environ 1 :200.

4. Utilisation selon l'une quelconque des revendications précédentes, dans laquelle le pH est dans la plage de 3,5 à 5,5.

5. Procédé pour contrôler les signes du vieillissement choisis dans le groupe constitué des ridules, des rides, de l'affaissement, du manque de tonicité et des taches de vieillesse, comprenant la fourniture d'une composition comprenant :
(i) d'environ 0,1 % à environ 30 % en poids d'un sel qui est un acide malonique neutralisé à l'ammoniac ;
(ii) d'environ 1 % à environ 99,9 % en poids d'un support cosmétiquement acceptable ; dans laquelle la composition a un pH dans la plage de 3 à 6,5.
